# EUROPEAN PATENT APPLICATION

(11) **EP 4 450 229 A1**
(43) Date of publication of application: **23.10.2024**
(21) Application number: 23895546.2
(22) Date of filing: 14.03.2023
(51) Int. Cl.: B25B 23/10

(54) **GRIP OPERATION TOOL, GRIP OPERATION TOOL SET USING SAME, AND ROBOT USING SAME**

(30) Priority: 03.03.2023 JP 2023032756
(71) Applicant: Medmetalex Co., Ltd, Nishiyodogawa-ku Osaka-shi, Osaka 555-0012 (JP); AMINO, Hirokazu, Takarazuka-shi, Hyogo 665-0061 (JP)
(72) Inventor: The designation of the inventor has not yet been filed
(74) Representative: VKK Patentanwälte PartG mbB
(86) International application number: PCT/JP2023/009811
(87) International publication number: WO 2024/185151

(57) **Abstract**

There is provided a holding operation tool such as a screwdriver that fastens and operates an engaged body such as a screw, wherein there is no risk of a screw falling from the holding operation tool such as a screwdriver when it is moved to a predetermined position.

A holding operation tool 1 fastens and operates an engaged body 10 including a fastened portion 11 to be fastened. The holding operation tool includes a handle 2 that operates the engaged body, shafts 4, 6 that protrude from the handle, a first fastening portion 3 that is engaged with the fastened portion and is disposed on a tip side of the shaft, and a second fastening portion 5 that is shaped so as to be incapable of being engaged with the fastened portion when the shaft is merely biased toward the tip side after the first fastening portion is engaged with the fastened portion, and so as to be engaged with the fastened portion when the handle is turned to twist the shaft by a predetermined angle and bias the shaft toward the tip side, and that is disposed on the shaft.

## Description

### [Technical Field]

The present invention relates to a holding operation tool for fastening and operating a engaged body, and a robot using the same.

### [Background Art]

An engaged body such as screws and bolts are installed or removed in various scenes. In some cases, screws are installed or removed during the manufacturing process of products or apparatuses in factories or the like. In other cases, engaged body are installed to set up equipment or facilities. In still other cases, screws, bolts, and the like are removed to replace or repair equipment or facilities. These operations are usually performed with a holding operation tool, such as a screwdriver.

When installing or removing a screw or the like with a screwdriver, the screw or the like is placed on the tip of the screwdriver. This applies to both a manually operated screwdriver and an electric screwdriver that rotates with electric power.

An engaged body, such as a screw, has a fastened portion, such as a groove or hole, in its head that can be turned by a screwdriver. The tip of the screwdriver is inserted into this fastened portion to install or remove the engaged body. At this point, the screwdriver and screw are not firmly connected because the tip of the screwdriver is only inserted into the groove or hole in the screw. Contact between the tip of the screwdriver and the groove or hole in the screw is maintained by the worker's hands holding the screw and screwdriver. Therefore, if the screwdriver and screw are difficult to hold or the grip force is insufficient, the screw may fall off the tip of the screwdriver. Several measures are taken to prevent this.

In the invention described in Patent Document 1 (see FIG. 26), a cross bit 503 is attached to the tip of a hexagonal shaft 504 of a screwdriver, a cylindrical chuck 502 is disposed around the cross bit 503, and a cylindrical holder 501 is provided outside of the cylindrical chuck 502. The distal end of the cylindrical chuck 502 is bent inward to form a claw 502a. When a screw is attached to the end portion of the cross bit 503, the screw is caught by the claw 502a so that the screw is less likely to fall off even when the screw is facing downward. However, since the screw is only caught by the claw 502a, the screw may fall off if it is subjected to a downward force (e.g., acceleration during motion).

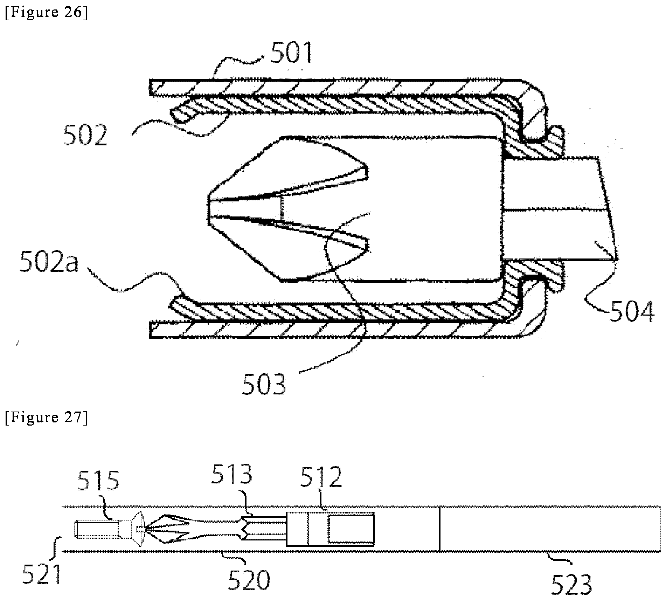

In the invention described in Patent Document 2 (see FIG. 27), a handgrip 523 of a screwdriver is provided with a cylindrical body 520, a movement part 512 is disposed in the cylindrical body 520, and a screw 515 is interlocked with the distal end of a tip portion 513 of the tip. The screw 515 does not fall in the direction shown in FIG. 27. However, it is easy to anticipate that the screw 515 will fall off when an aperture 521 is facing downward.

In the invention described in Patent Document 3 (see FIG. 28), a tip portion 603 is provided at the distal end of a shank coupled to a handle 610 of a screwdriver, and a fitting part 607 is disposed in the middle of the shank. The fitting part 607 includes an annular support 613 and an annular part 611, and a magnetic part 609 is attached to the distal end of the annular part 611. When the fitting part 607 is moved to a flange 617, the tip portion 603 is magnetized. If a screw interlocked with the tip portion 603 is made of a magnetic material, the screw is magnetized and locked by magnetic force and is unlikely to fall off. However, it is easy to anticipate that the screw will fall off if the screwdriver is subjected to acceleration or impact during movement. In addition, a non-magnetized stainless steel screwdriver does not provide drop protection.

In the invention described in Patent Document 4 (see FIG. 29), a screwdriver 701 having a grip 703 and a main body 702 is provided, and a tip portion 704 at the distal end of the main body 702 has concave parts 742 and a convex part 741. Adsorptive flexible members 705 are attached to the concave parts 742. These flexible members 705 enter into the locking hole or groove in a screw to make the screw less prone to falling off. However, since the mechanism for making the screw less prone to falling off is friction between the adsorptive flexible members 705 and the groove or hole in the screw, it is easily anticipated that the screw will fall off if the screwdriver is subjected to acceleration or impact during movement.

In the invention described in Patent Document 5 (see FIG. 30), a screwdriver 832 has, at the distal end, a first blade 721, a second blade 722, a third blade 723, a fourth blade 724, a hollow 913, a hollow 923, a projection 911, and a projection 921. When the tip of the screwdriver is pressed into the groove or hole in a screw, the hollow 913 and the hollow 923 become smaller, the projection 911 and the projection 921 are brought into pressure contact with the groove or hole in the screw, and the first blade 721 and the second blade 722 move to expand so that the screw becomes less prone to fall off. However, the force of pressing the screwdriver tip into the groove or hole in the screw varies, and the screw may fall off the screwdriver if the pressing force is weak.

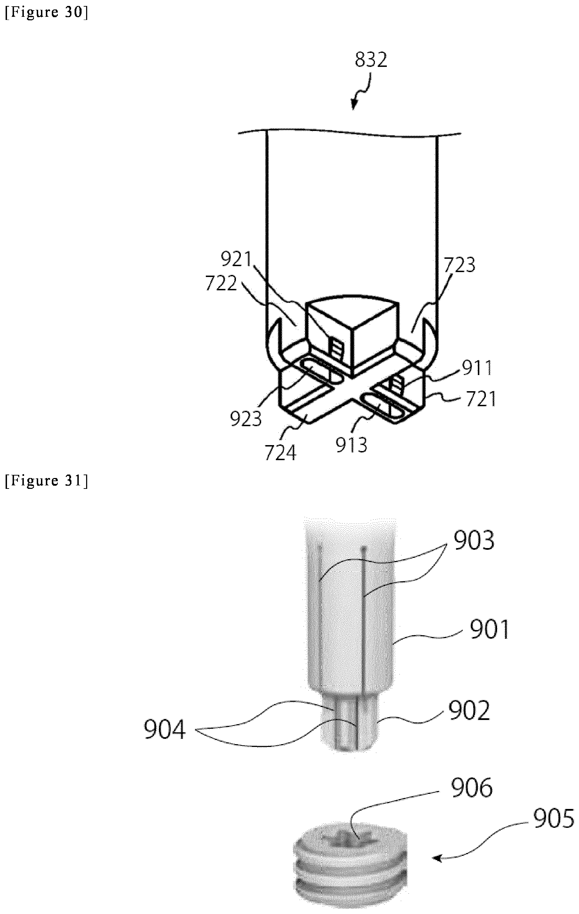

A screw 905 and a screwdriver as shown in FIG. 31 are used for vertebral surgery. A shank 901 of the screwdriver has slits 903, and a tip portion 902 has slits 904. The screw 905 has a locking hole (Torx^{®}) 906, and the tip portion 902 is made slightly larger than the hole 906. Because of the slits 903 and the slits 904, the tip of the screwdriver enters into the hole 906 when the tip of the screwdriver is pressed into the hole 906, and the screw 905 is less likely to fall off the screwdriver due to the expanding force of the tip portion. However, since the screw is locked only by the expanding force of the tip, it is easy to anticipate that the screw will fall off if the screwdriver is subjected to acceleration or impact during movement. In addition, using the screwdriver to apply a large amount of torque to tighten the screw may break the tip of the screwdriver.

Instead of using the slots in FIG. 31, another method is used in which the tip portion 902 is tapered and mated with the screw 905 and an impact is applied to the tip portion 902 to engage the screw 905. However, this method is not reliable because the applied impact force varies in strength.

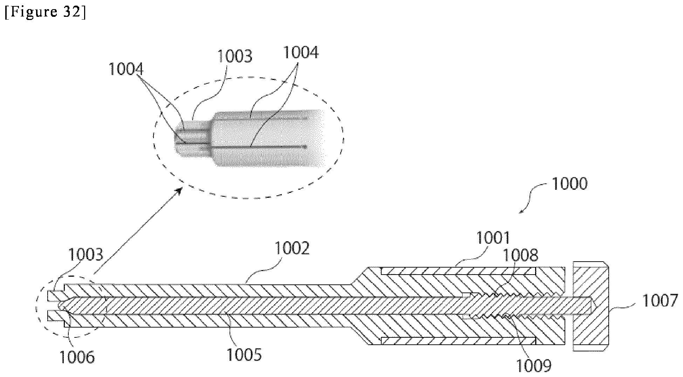

A holding operation tool 1000 shown in FIG. 32 consists of a handle 1001, a shank 1002, a tip portion 1003, a rod 1005, a knob 1007, and the like. A rod tip portion 1006 has slits 1004 like the screwdriver in FIG. 31. In the holding operation tool 1000 of FIG. 32, as the knob 1007 is rotated further, a male thread 1008 and a female thread 1009 engage to move the rod tip portion 1006 to the left and to push and widen the tip portion 1003. By pushing and widening the tip portion, the screw can be firmly tightened. However, as with the screwdriver shown in FIG. 31, using the screwdriver to apply a large amount of torque to tighten the screw may break the tip of the screwdriver.

### [Prior Art Documents]

### [Patent Documents]

[Patent Literature 1] Japanese Registered Utility Model No. 3131577
[Patent Literature 2] Japanese Laid-Open Patent Publication No. 2010-137332
[Patent Literature 3] Japanese Laid-Open Patent Publication No. 2010-42486
[Patent Literature 4] Japanese Laid-Open Patent Publication No. 2020-62713
[Patent Literature 5] Japanese Laid-Open Patent Publication No. 2020-66091

### [Summary of the Invention]

### [Problems to be Solved by the Invention]

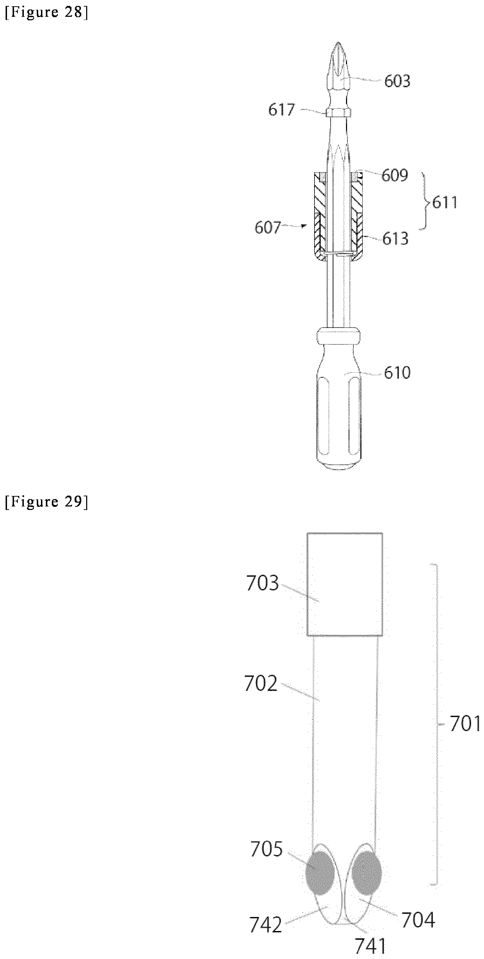

In the conventional screwdrivers shown in FIGS. 26 to 32, the screw interlocked with the tip of the screwdriver may become detached and fall off due to impact or the like, and it is not ensured that the screw will not fall off until the screwdriver starts screwing. For example, many screws are used to place implants in orthopedic and spinal surgeries. In such cases, if a screw falls into the surgical field of a patient undergoing surgery, the screw may enter the patient's body, resulting in a medical accident. In other industries, the risk of a engaged body such as a screw falling off a screwdriver can also be a source of inefficiency and inconvenience.

The present invention has been conceived in view of such circumstances, and an object of the present invention is to provide a holding operation tool such as a screwdriver that fastens and operates a engaged body such as a screw, wherein there is no risk of a screw falling from the holding operation tool such as a screwdriver when it is moved to a predetermined position.

### [Means to Solve the Problem]

The holding operation tool according to the present invention for solving the problems described above has the function of holding a engaged body such as a screw interlocked with the tip so as not to separate from the tip during movement of the holding operation tool, which is a novel function compared to conventional holding operation tools such as a screwdriver which are not reliable in this respect.

An aspect (1) of the present invention is a holding operation tool that fastens and operates a engaged body including a fastened portion to be fastened, wherein the holding operation tool includes a handle that operates the engaged body, a shaft that protrudes from the handle, a first fastening portion that is engaged with the fastened portion and is disposed on a tip side of the shaft, and a second fastening portion that is shaped so as to be incapable of being engaged with the fastened portion when the shaft is merely moved to the tip side after the first fastening portion is engaged with the fastened portion, and so as to be engaged with the fastened portion when the shaft is twisted by a predetermined angle and is moved to the tip side, and that is disposed on the shaft.

The holding operation tool is a tool that fastens and operates a engaged body such as a screw, a bolt, or a vis. For example, a screwdriver performs its function by placing a engaged body, such as a bolt, on the tip and tightening it. A engaged body, such as a screw, has a fastened portion in its head that engages with the screwdriver. The fastened portion takes various forms, such as a minus groove, a plus groove, a hexagonal hole, a Torx hole, and a bolt head.

In aspect (1) of the present invention, the holding operation tool includes the handle, the shaft that protrudes from the handle, the first fastening portion that is engaged with the fastened portion and is disposed on the tip side of the shaft, and the second fastening portion that is shaped so as to be incapable of being engaged with the fastened portion when the shaft is merely moved to the tip side after the first fastening portion is engaged with the fastened portion, and so as to be engaged with the fastened portion when the shaft is twisted by a predetermined angle and is moved to the tip side, and that is disposed on the shaft, so that the engaged body does not come off from the tip of the holding operation tool during operation.

Aspect (2) of the present invention is the holding operation tool of aspect (1) of the present invention, wherein the shaft has a first shaft that is coupled to the first fastening portion and a second shaft that is coupled to the second fastening portion, the first shaft and the second shaft are concentric, one of the first shaft and the second shaft has a cylindrical body through which the other passes, and the both shafts are coupled to the handle.

In aspect (2) of the present invention, the shaft has the first shaft that is coupled to the first fastening portion and the second shaft that is coupled to the second fastening portion, the first shaft and the second shaft are concentric, one of the first shaft and the second shaft has a cylindrical body through which the other passes, the both shafts are coupled to the handle, and the two shafts, the first shaft coupled to the first fastening portion and the second shaft coupled to the second fastening portion, are provided so that the variation of the means for fastening the engaged body increases and the engaged body does not come off from the tip of the holding operation tool during operation.

Aspect (3) of the present invention is the holding operation tool of aspect (1) of the present invention, wherein the shaft has a first shaft that is coupled to the first fastening portion and a second shaft that is coupled to the second fastening portion, the first shaft and the second shaft are concentric, one of the first shaft and the second shaft has a cylindrical body through which the other passes, and one shaft of the first shaft and the second shaft is coupled to the handle, and the other shaft is coupled to the one shaft.

In aspect (3) of the present invention, the shaft has the first shaft that is coupled to the first fastening portion and the second shaft that is coupled to the second fastening portion, the first shaft and the second shaft are concentric, one of the first shaft and the second shaft has a cylindrical body through which the other passes, and one shaft of the first shaft and the second shaft is coupled to the handle, and the other shaft is coupled to the one shaft so that the option of the means for fixing the first shaft and the second shaft increases, and the engaged body does not come off from the tip of the holding operation tool during operation.

Aspect (4) of the present invention is the holding operation tool of any one of aspects (1) to (3) of the present invention, wherein the fastened portion is a recess formed in the **engaged body,** and the first fastening portion and the second fastening portion engage with the recess.

In aspect (4) of the present invention, the fastened portion is the recess formed in the engaged body, and the first fastening portion and the second fastening portion engage with the recess so that the engaged body needs only the recess as the fastened portion and the engaged body does not come off from the tip of the holding operation tool during operation even if the fastened portion of the engaged body is of a simple structure.

Aspect (5) of the present invention is the holding operation tool of any one of aspects (1) to (3) of the present invention, wherein the fastened portion is an outer side surface formed on the engaged body, and the first fastening portion and the second fastening portion are engaged with the outer side surface.

In aspect (5) of the present invention, the fastened portion is the outer side surface formed on the engaged body, and the first fastening portion and the second fastening portion are engaged with the outer side surface so that the engagement can be performed by using the outer side surface, and the engaged body does not come off from the tip of the holding operation tool during operation even if a recess cannot be provided in the engaged body as in aspect (5) of the present invention.

Aspect (6) of the present invention is the holding operation tool of any one of aspects (1) to (3) of the present invention, wherein the fastened portion is a recess formed in the engaged body and is an outer side surface formed on the engaged body, one of the first fastening portion and the second fastening portion is engaged with the recess, and the other fastening portion is engaged with the outer side surface.

In aspect (6) of the present invention, the fastened portion is the recess formed in the engaged body and is the outer side surface formed on the engaged body, one of the first fastening portion and the second fastening portion is engaged with the recess, and the other fastening portion is engaged with the outer side surface so that the recess and the outer side surface can be used as the fastened portion. Accordingly, the engaged body does not come off from the tip of the holding operation tool during operation.

An aspect (7) of the present invention is a holding operation tool set including engaged body including a fastened portion to be fastened, a holding operation tool that fastens and operates the engaged body, the holding operation tool including a handle that operates the engaged body, a shaft that protrudes from the handle, a first fastening portion that is engaged with the fastened portion and is disposed on a tip side of the shaft, and a second fastening portion that is shaped so as to be incapable of being engaged with the fastened portion when the shaft is merely biased toward the tip side after the first fastening portion is engaged with the fastened portion, and so as to be engaged with the fastened portion when the handle is turned to twist the shaft by a predetermined angle and bias the shaft toward the tip side, and that is disposed on the shaft, and a base plate that detachably holds the engaged body.

An aspect (8) of the present invention is a robot that fastens and operates a engaged body including a fastened portion to be fastened, the robot including a robot main body, an arm portion that is connected to the robot main body, a controller that controls the arm portion, and a holding operation tool that fastens the engaged body at a tip of the arm portion, wherein the holding operation tool includes a handle that operates the engaged body, a shaft that protrudes from the handle, a first fastening portion that is engaged with the fastened portion and is disposed on a tip side of the shaft, and a second fastening portion that is shaped so as to be incapable of being engaged with the fastened portion when the shaft is merely moved to the tip side after the first fastening portion is engaged with the fastened portion, and so as to be engaged with the fastened portion when the shaft is twisted by a predetermined angle and is moved to the tip side, and that is disposed on the shaft.

When the holding operation tool is attached to the tip of a robot arm, there is no risk of a engaged body (a screw or the like) falling off the tip of the robot arm, even when the robot is moving at a high speed and undergoing high acceleration. This makes it possible to fully automate an assembly process in the production line of vehicles, for example, where various screws such as bolts are used.

### [Effect of the invention]

According to the present invention, there can be provided a holding operation tool such as a screwdriver that fastens and operates an engaged body such as a screw, wherein there is no risk of a screw falling from the holding operation tool such as a screwdriver when it is moved to a predetermined position.

### [Brief description of the drawings]

FIG. 1 is a perspective view of a holding operation tool (screwdriver) in a first embodiment of the present invention.
FIG. 2 is a partially schematic cross-sectional view of the screwdriver in the first embodiment of the present invention.
FIG. 3 is a schematic diagram illustrating a state in which a screw is held and moved by using the screwdriver in the first embodiment of the present invention.
FIG. 4 is a partially schematic cross-sectional view of a screwdriver in a second embodiment of the present invention.
FIG. 5 is a schematic diagram illustrating a state in which a screw is held and moved by using the screwdriver of the second embodiment of the present invention.
FIG. 6 is a partially schematic cross-sectional view of a screwdriver in a third embodiment of the present invention.
FIG. 7 is a partially schematic cross-sectional view of a screwdriver in a fourth embodiment of the present invention.
FIG. 8 is a schematic diagram illustrating a state in which a screw is held and moved by using the screwdriver of the fourth embodiment of the present invention.
FIG. 9 is a partially schematic cross-sectional view of a screwdriver in a fifth embodiment of the present invention.
FIG. 10 is a schematic diagram illustrating a state in which a screw is held and moved by using the screwdriver of the fifth embodiment of the present invention.
FIG. 11 is a partially schematic cross-sectional view of a screwdriver in a sixth embodiment of the present invention.
FIG. 12 is a schematic diagram illustrating a screw/screwdriver set of the present invention.
FIG. 13 is a modification example of the screwdriver of the first embodiment shown in FIG. 2.
FIG. 14 is a modification example of the screwdriver of the fourth embodiment shown in FIG. 7.
FIG. 15 is a partially schematic cross-sectional view of a holding operation tool for a hip stem in a seventh embodiment of the present invention.
FIG. 16 is a partially schematic cross-sectional view of a holding operation tool for an acetabulum shell in an eighth embodiment of the present invention.
FIG. 17 is a schematic perspective view of a shaft, a fastening portion, and a engaged body in a ninth embodiment of the present invention.
FIG. 18 is a schematic diagram illustrating examples of relationships between various holding operation tools and various engaged body of the present invention.
FIG. 19 is a perspective view of a flexible screwdriver in a tenth embodiment of the present invention.
FIG. 20 is a perspective view of an acetabular reamer cup holder in an eleventh embodiment of the present invention.
FIG. 21 is a perspective view of a stem holder in a twelfth embodiment of the present invention.
FIG. 22 is a perspective view of a stem holder in a thirteenth embodiment of the present invention.
FIG. 23 is a perspective view of a screwdriver in a fourteenth embodiment of the present invention.
FIG. 24 is a perspective view of a hexagonal wrench in a fifteenth embodiment of the present invention.
FIG. 25 is a schematic view of a robot in a sixteenth embodiment that has the holding operation tool of the first embodiment of the present invention at the tip of the arm.
FIG. 26 is a partial cross-sectional view of a screwdriver tip described in Patent Document 1.
FIG. 27 illustrates a screwdriver described in Patent Document 2.
FIG. 28 is a partial cross-sectional view of a screwdriver described in Patent Document 3.
FIG. 29 illustrates a screwdriver described in Patent Document 4.
FIG. 30 is a partial cross-sectional view of a screwdriver tip described in Patent Document 5.
FIG. 31 is a perspective view of a tip portion of a screwdriver and a screw used in conventional spine surgery.
FIG. 32 illustrates a screwdriver used in conventional spine surgery, which is an improved type of the screwdriver in FIG. 30.

### [Description of Embodiments]

Hereinafter, embodiments of the present invention will be described in detail with reference to the drawings. The following embodiments are essentially preferred examples and are not intended to limit the present invention, the objects to which the present invention is applied, or the scope of the present invention.

### First Embodiment

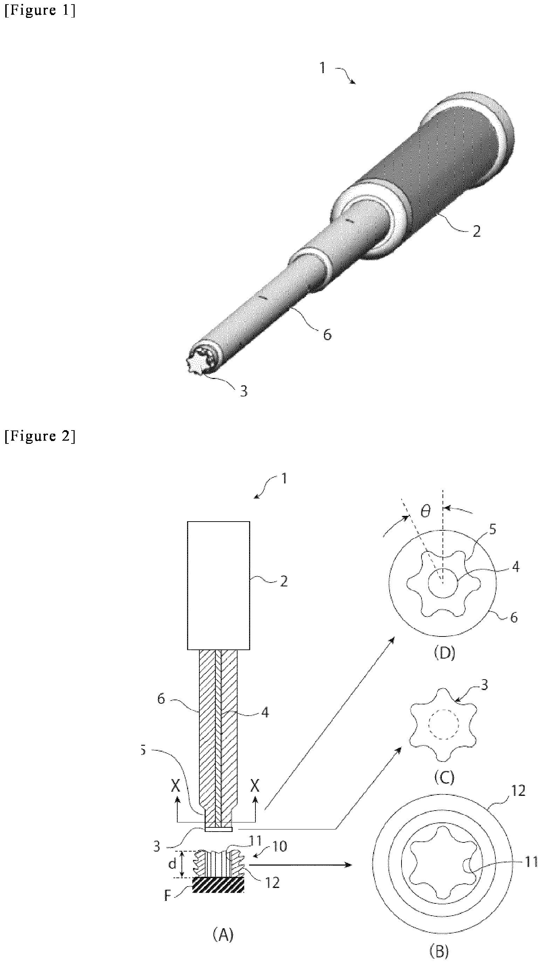

FIGS. 1 to 3 illustrate a first embodiment of a holding operation tool of the present invention.

A holding operation tool 1 illustrated in FIG. 1 includes a handle 2, a first fastening portion 3, and a second shaft 6.

FIG. 2 is a partially schematic cross-sectional view of the holding operation tool in the first embodiment. FIG. 2A illustrates the handle 2 to be gripped by the user, the first fastening portion 3, a first shaft 4 that couples the handle 2 and the first fastening portion 3, and the second shaft 6 through which the first shaft 4 passes. A second fastening portion 5 at the tip of the second shaft 6 is not coupled to the first fastening portion 3, but is coupled only to the handle 2.

A engaged body 10 is detachably disposed on a fixture (base plate) F. The engaged body 10 has a male thread 12 cut therein and has a fastened portion 11 passing through the center. The depth of the hole in the fastened portion 11 is a predetermined depth d. FIG. 2B is a top view of the engaged body 10 in which the fastened portion 11 has a Torx shape. FIG. 2C is a bottom view of the first fastening portion 3 shaped to mate with the fastened portion 11. The shape of the first fastening portion 3 does not need to coincide with the shape of the fastened portion 11 and may have a Torx shape in which, for example, a projection is partially chipped. FIG. 2D is a cross-sectional view of the second fastening portion 5 along the line X-X in which the outer periphery of the second fastening portion 5 also has a Torx shape but is shifted by an angle θ from the Torx shape of the first fastening portion 3. Therefore, even if an attempt is made to mate the holding operation tool 1 with the engaged body 10, the second fastening portion 5 does not enter the fastened portion 11 so that the engaged body 10 cannot be fastened.

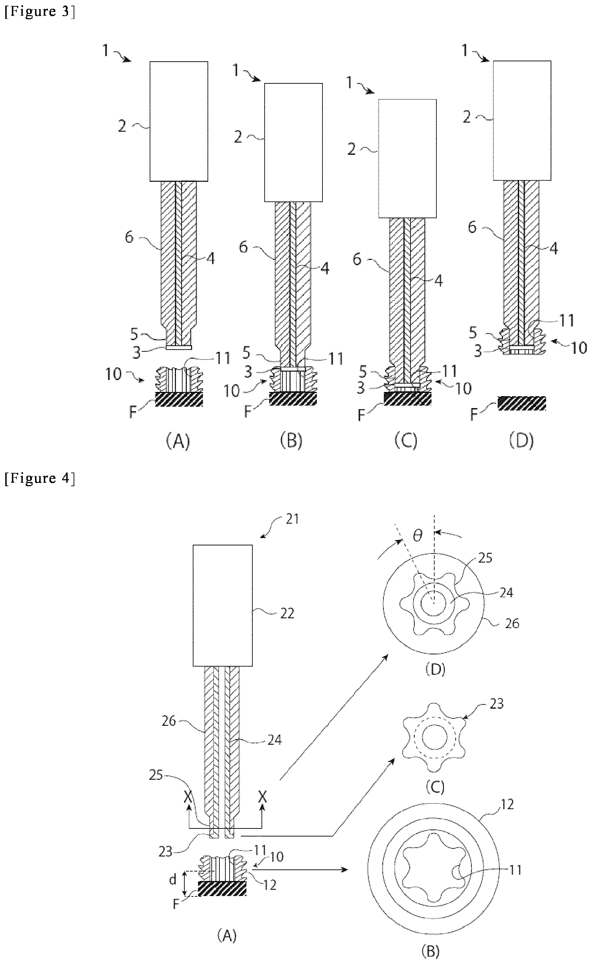

FIG. 3 is a schematic diagram illustrating a state in which a screw is held and moved by using the screwdriver of the first embodiment. FIG. 3A illustrates the state as shown in FIG. 2, and FIG. 3B illustrates a state in which the first fastening portion 3 is mated with the fastened portion 11. Since the shape of the second fastening portion 5 is shifted by the angle θ from the first fastening portion 3, even if an attempt is made to press the second fastening portion 5 into the fastened portion 11 by applying a downward force to the handle 2, the second fastening portion 5 cannot enter the fastened portion 11.

When the handle 2 is turned from the state shown in FIG. 3B to apply torque, the angle phase difference θ between the Torx shapes of the second fastening portion 5 and the first fastening portion 3 becomes small. When the phase difference disappears, the second fastening portion 5 enters the fastened portion 11 to bring about the state shown in FIG. 3C. When the handle 2 is moved upward from this state, the engaged body 10 is firmly held by the holding operation tool 1 as shown in FIG. 3D, so that it is possible to reliably prevent the engaged body 10 from coming off the holding operation tool 1.

### Second Embodiment

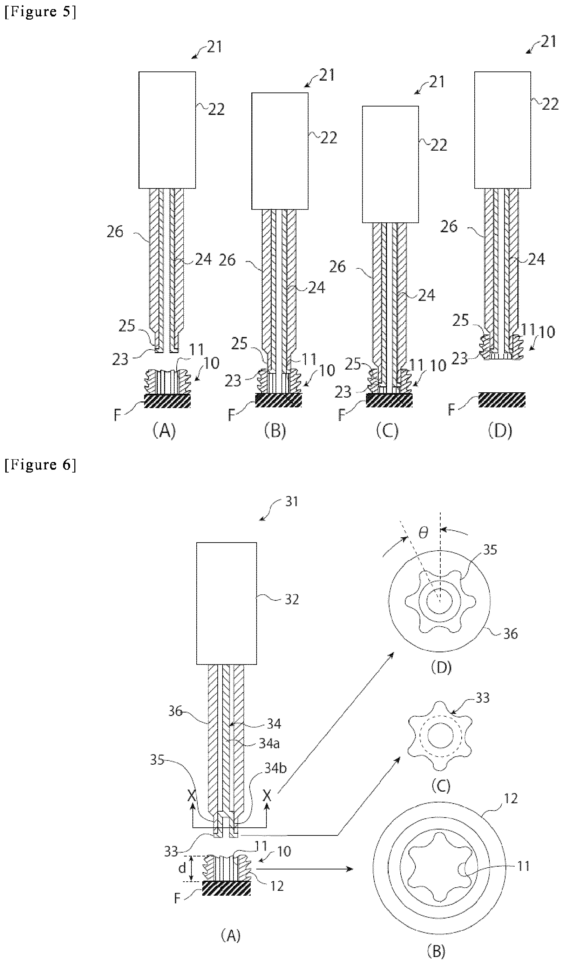

FIGS. 4 and 5 illustrate a second embodiment of a screwdriver of the present invention.

FIG. 4 is a partially schematic cross-sectional view of a holding operation tool 21 in the second embodiment. FIG. 4A illustrates a handle 22 to be gripped by the user, a first fastening portion 23, a first shaft 24 that couples the handle 22 and the first fastening portion 23, and a second shaft 26 through which the first shaft 24 passes. A second fastening portion 25 at the tip of the second shaft 26 is not coupled to the first fastening portion 23, but is coupled only to the handle 22.

A engaged body 10 shown in FIG. 4A is the same as the engaged body 10 shown in FIG. 2, and therefore the description thereof is omitted. FIGS. 4B-4D are also the same as FIGS. 2B-2D, and therefore the description thereof is omitted.

FIG. 5 is a schematic diagram illustrating a state in which a screw is held and moved by using the screwdriver of the second embodiment. FIG. 5A illustrates the state as shown in FIG. 4, and FIG. 5B illustrates a state in which the first fastening portion 23 is mated with the fastened portion 11. Since the shape of the second fastening portion 25 is shifted by an angle θ from the first fastening portion 23, even if an attempt is made to press the second fastening portion 25 into the fastened portion 11 by applying a downward force to the handle 22, the second fastening portion 25 cannot enter the fastened portion 11.

When the handle 22 is turned from the state shown in FIG. 5B to apply torque, the angle phase difference θ between the Torx shapes of the second fastening portion 25 and the first fastening portion 23 becomes small. When the phase difference disappears, the second fastening portion 25 enters the fastened portion 11 to bring about the state shown in FIG. 5C. When the handle 22 is moved upward from this state, the engaged body 10 is firmly held by the holding operation tool 21 as shown in FIG. 5D, so that it is possible to reliably prevent the engaged body 10 from coming off the holding operation tool 21.

### Third Embodiment

FIG. 6 illustrates a third embodiment of a screwdriver of the present invention.

FIG. 6 is a partially schematic cross-sectional view of a holding operation tool 31 in the third embodiment. FIG. 6A illustrates a handle 32 to be gripped by the user, a first fastening portion 33, and a first shaft 34 that couples the handle 32 and the first fastening portion 33. The first shaft 34 includes a rod 34a and a bottomed cylindrical body 34b at the distal end of the rod 34a. A second shaft 36 is provided through which the first shaft 34 passes. A second fastening portion 35 at the tip of the second shaft 36 is not coupled to the first fastening portion 33, but is coupled only to the handle 32.

An engaged body 10 shown in FIG. 6A is the same as the engaged body 10 shown in FIG. 2, and therefore the description thereof is omitted. FIGS. 6B-6D are also the same as FIGS. 2B-2D, and therefore the description thereof is omitted.

### Fourth Embodiment

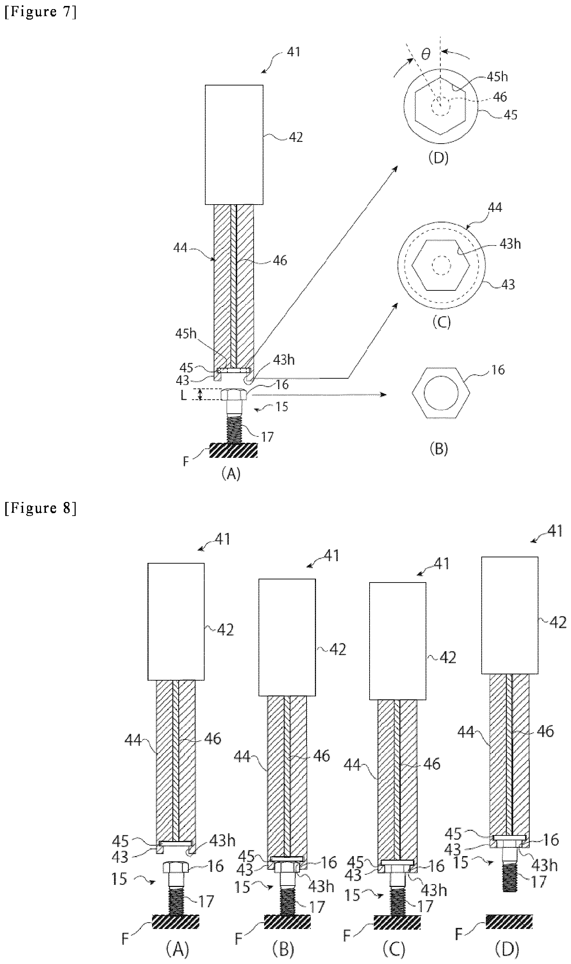

FIGS. 7 and 8 illustrate a fourth embodiment of a screwdriver of the present invention.

FIG. 7 is a partially schematic cross-sectional view of a holding operation tool 41 in the fourth embodiment. FIG. 7A illustrates a handle 42 to be gripped by the user, a first fastening portion 43, a first shaft 44 that couples the handle 42 and the first fastening portion 43, and a second shaft 46 that passes through the first shaft 44.

A second fastening portion 45 at the tip of the second shaft 46 is not coupled to the first shaft 44, but is coupled only to the handle 42. The first fastening portion 43 has a hole 43h for engaging with (an outer side surface of) a fastened portion 16. The second fastening portion 45 has a hole 45h for engaging with (the outer side surface of) the fastened portion 16.

An engaged body 15 is detachably disposed on a fixture (base plate) F. The engaged body 15 has a male thread 17 cut therein and has the fastened portion 16 (the outer side surface) formed on the head. The axial length of the fastened portion 16 is a predetermined axial length L. FIG. 7B is a top view of the engaged body 15 in which the fastened portion 16 is hexagonal in shape. FIG. 7C is a bottom view of the first fastening portion 43 shaped to mate with the fastened portion 16. The shape of the first fastening portion 43 does not need to coincide with the shape of the fastened portion 16. For example, the first fastening portion 43 may have a partially deformed hexagonal shape as far as the first fastening portion 43 can be engaged with the fastened portion 16. FIG. 7D is a bottom view of the second fastening portion 45 in which the hole 45h of the second fastening portion 45 is also hexagonal in shape but is shifted at an angle θ from the hexagonal shape of the hole 43h. Therefore, even if an attempt is made to mate the holding operation tool 41 with the engaged body 15, the fastened portion 16 cannot be engaged with the second fastening portion 45 so that the engaged body 15 cannot be fastened.

FIG. 8 is a schematic diagram illustrating a state in which a screw is held and moved by using the holding operation tool 41 of the fourth embodiment. FIG. 8A illustrates the state as shown in FIG. 7, and FIG. 8B illustrates a state in which the first fastening portion 43 is mated with the fastened portion 16. Since the shape of the second fastening portion 45 is shifted by the angle θ from the first fastening portion 43, even if an attempt is made to press the second fastening portion 45 into the fastened portion 16 by applying a downward force to the handle 42, the second fastening portion 45 cannot be engaged with the fastened portion 16.

When the handle 42 is turned from the state shown in FIG. 8B to apply torque, the angle phase difference θ in the hexagonal shape of the second fastening portion 45 becomes small. When the phase difference disappears, the second fastening portion 45 is engaged with the fastened portion 16 to bring about the state shown in FIG. 8C. When the handle 42 is moved upward from this state, the engaged body 15 is firmly held by the holding operation tool 41 as shown in FIG. 8D, so that it is possible to reliably prevent the engaged body 15 from coming off the holding operation tool 41.

### Fifth Embodiment

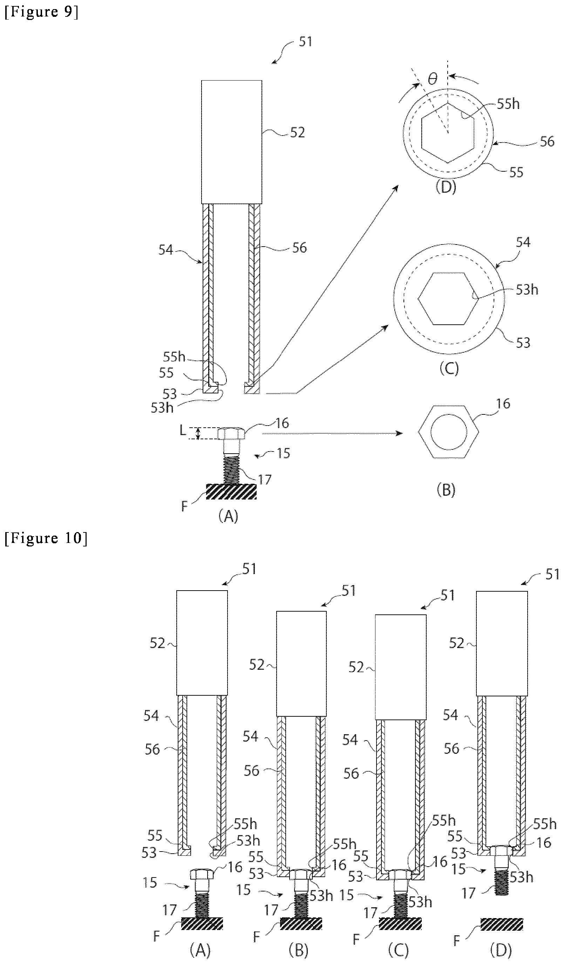

FIGS. 9 and 10 illustrate a fifth embodiment of a screwdriver of the present invention.

FIG. 9 is a partially schematic cross-sectional view of a holding operation tool 51 in the fifth embodiment. FIG. 9A illustrates a handle 52 to be gripped by the user, a first fastening portion 53, a first shaft 54 that couples the handle 52 and the first fastening portion 53, and a second shaft 56 that passes through the first shaft 54. A second fastening portion 55 at the tip of the second shaft 56 is not coupled to the first shaft 54, but is coupled only to the handle 52. The first fastening portion 53 has a hole 53h for engaging with a fastened portion 16. The second fastening portion 55 has a hole 55h for engaging with the fastened portion 16.

An engaged body 15 shown in FIG. 9A is the same as the engaged body 15 shown in FIG. 7, and therefore the description thereof is omitted. FIGS. 9B-9D are also the same as FIGS. 7B-7D, and therefore the description thereof is omitted.

FIG. 10 is a schematic diagram illustrating a state in which the engaged body 15 is held and moved by using the holding operation tool 51 of the fifth embodiment. FIG. 10A illustrates the state as shown in FIG. 9, and FIG. 10B illustrates a state in which the hole 53h is engaged with the fastened portion 16. Since the shape of the hole 55h is shifted by an angle θ from the first fastening portion 53, even if an attempt is made to press the second fastening portion 55 into the fastened portion 16 by applying a downward force to the handle 52, the hole 55h cannot be engaged with the fastened portion 16.

When the handle 52 is turned from the state shown in FIG. 10B to apply torque, the angle phase difference θ in the hexagonal shape of the hole 55h becomes small. When the phase difference disappears, the second fastening portion 55 is engaged with the fastened portion 16 to bring about the state shown in FIG. 10C. When the handle 52 is moved upward from this state, the engaged body 15 is firmly held by the holding operation tool 51 as shown in FIG. 10D, so that it is possible to reliably prevent the engaged body 15 from coming off the holding operation tool 51.

### Sixth Embodiment

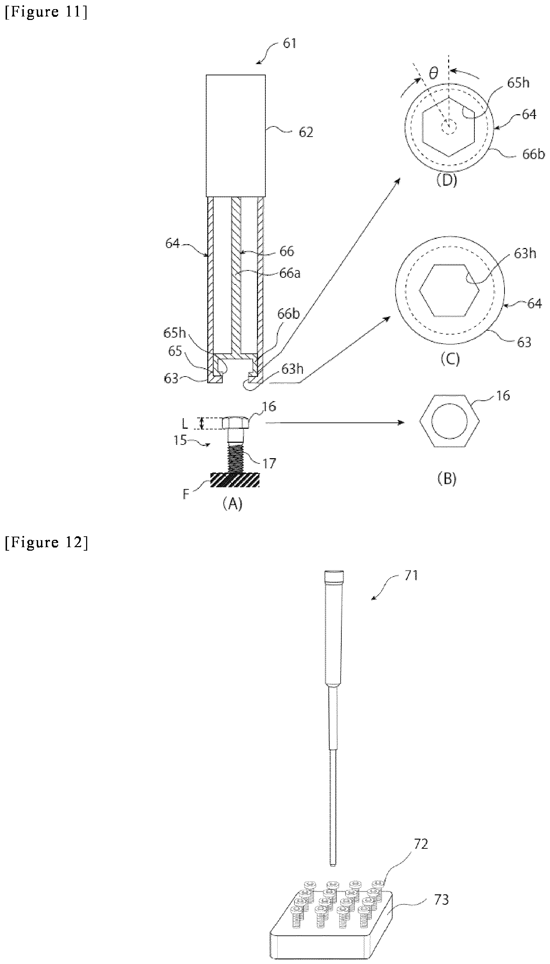

FIG. 11 illustrates a sixth embodiment of a screwdriver of the present invention.

FIG. 11 is a partially schematic cross-sectional view of a holding operation tool 61 in the sixth embodiment. FIG. 11A illustrates a handle 62 to be gripped by the user, a first fastening portion 63, and a first shaft 64 that couples the handle 62 and the first fastening portion 63. A second shaft 66 that passes through the first shaft 64 is provided. The second shaft 66 includes a rod 66a and a bottomed cylindrical body 66b at the distal end of the rod 66a. A second fastening portion 65 at the tip of the second shaft 66 is not coupled to the first shaft 64, but is coupled only to the handle 62. The first fastening portion 63 has a hole 63h for engaging with (an outer side surface of) a fastened portion 16. The second fastening portion 65 has a hole 65h for engaging with (the outer side surface of) the fastened portion 16.

A engaged body 15 shown in FIG. 11A is the same as the engaged body 15 shown in FIG. 7, and therefore the description thereof is omitted. FIGS. 11B-11D are also the same as FIGS. 7B-7D, and therefore the description thereof is omitted.

FIG. 12 illustrates a screw/screwdriver set including a screwdriver 71, screws 72 that are attached to the tip of the screwdriver 71 when in use, and a base plate 73 on which the screws 72 are detachably held. The screws 72 may be screwed to the base plate 73. Preparing such a set is effective when a very large number of screws are to be used and the screws 72 should not come off from the tip of the screwdriver 71 during use.

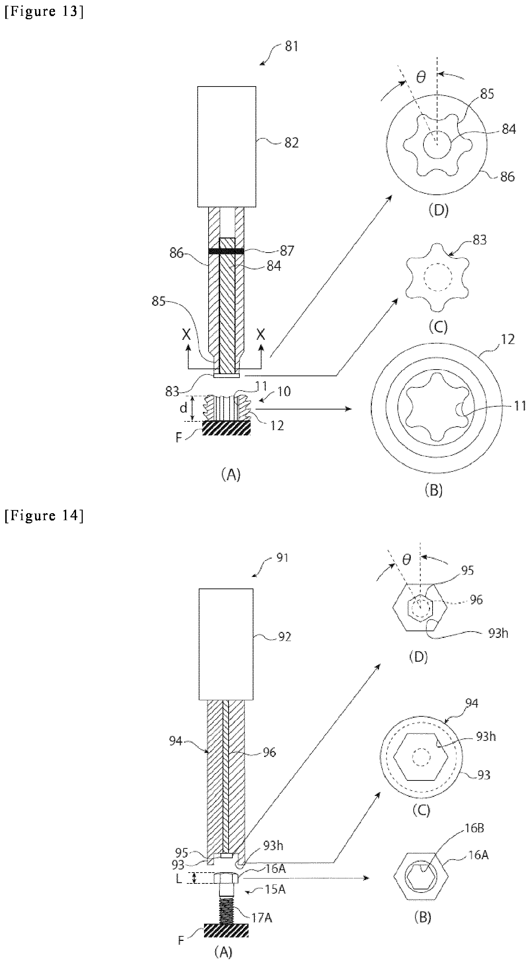

FIG. 13 illustrates a modification example of the screwdriver of the first embodiment shown in FIG. 2. FIG. 13A illustrates a holding operation tool 81 that includes a handle 82 to be gripped by the user, a first fastening portion 83, a first shaft 84 that couples the handle 82 and the first fastening portion 83, and a second shaft 86 through which the first shaft 84 passes. A second fastening portion 85 at the tip of the second shaft 86 is not coupled to the first fastening portion 83, but is coupled only to the handle 82. The first shaft 84 is not coupled to the handle 82 but is coupled to the second shaft 86 by a retaining pin 87. With this structure, the first shaft 84 and the second shaft 86 can be coupled without intervention of the handle 82.

An engaged body 10 and FIGS. 13B, 13C and 13D are the same as described above with reference to FIG. 2, and therefore the description thereof is omitted.

FIG. 14 illustrates a modification example of the screwdriver of the fourth embodiment shown in FIG. 7. FIG. 14A illustrates a handle 92 to be gripped by the user, a first fastening portion 93, a first shaft 94 that couples the handle 92 and the first fastening portion 93, and a second shaft 96 that passes through the first shaft 94.

A second fastening portion 95 at the tip of the second shaft 96 is not coupled to the first shaft 94, but is coupled only to the handle 92. The first fastening portion 93 has a hole 93h for engaging with an outer side surface 16A of a engaged body 15A. The second fastening portion 95 is engaged in a recess 16B formed in the head of the engaged body 15A.

The engaged body 15A is detachably disposed on a fixture (base plate) F. The engaged body 15A has a male thread 17A cut therein and has the outer side surface 16A that is the fastened portion formed on the head. The axial length of the outer side surface 16A is a predetermined axial length L. FIG. 14B is a top view of the engaged body 15A in which the outer side surface 16A is hexagonal in shape. FIG. 14C is a bottom view of the first fastening portion 93 that has a hole 93h for mating with the outer side surface 16A. FIG. 14D is a bottom view of the second fastening portion 95, that is hexagonal in shape to mate with the recess 16B, but shifted by an angle θ from the hexagonal shape of the recess 16B. Therefore, even if an attempt is made to mate the holding operation tool 91 with the recess 16B, the second fastening portion 95 does not enter the recess 16B so that the engaged body 15A cannot be fastened.

A series of operations of the holding operation tool 91 for holding and operating the engaged body 15A shown in FIG. 14 are also the same as those described with reference to FIG. 8, and therefore the description thereof is omitted.

### Seventh Embodiment

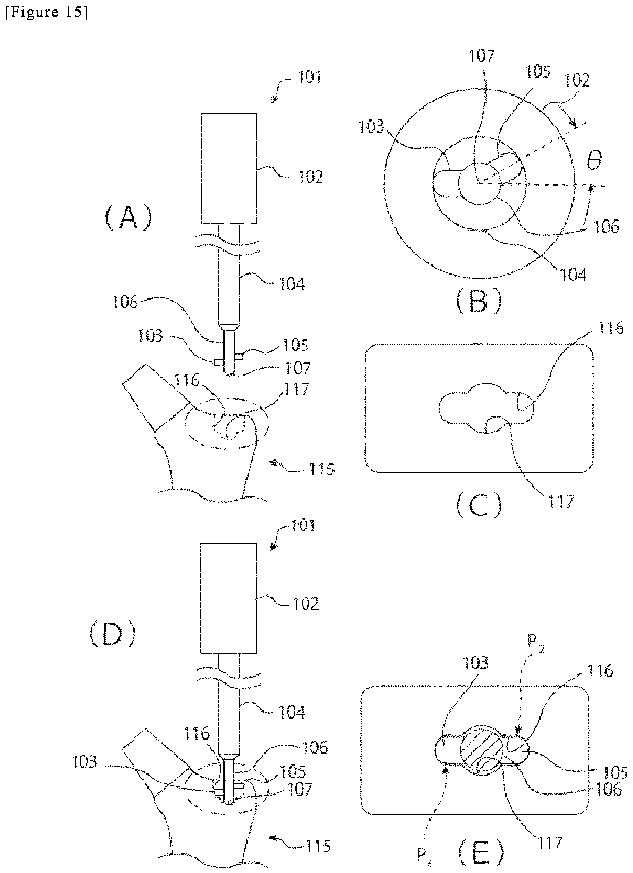

FIG. 15 is a partially schematic cross-sectional view of a holding operation tool for a hip stem of a seventh embodiment of the present invention. As shown in FIG. 15A, a holding operation tool 101 includes a handle 102, a shaft 104, a shaft small diameter portion 106, a first fastening portion 103, a second fastening portion 105, and a tip projection 107. A engaged body 115 is a hip stem. On the shoulder of the engaged body 115, a fastened portion 116 and a bottom hole 117 are provided for engaging and driving. The seventh embodiment differs from the embodiments described above in that the first fastening portion 103 and the second fastening portion 105 are coupled to the shaft small diameter portion 106, that is a shaft connected to the shaft 104.

FIG. 15B is a bottom view of the holding operation tool 101. The first fastening portion 103 and the second fastening portion 105 are shifted by an angle θ from each other, and the second fastening portion 105 cannot fit into the fastened portion 116 shown in FIG. 15C. In the state in which the first fastening portion 103 fits into the fastened portion 116, the handle 102 is twisted to twist the shaft small diameter portion 106 and reduce the angle θ. Then, when the handle 102 is pressed toward the tip side, the second fastening portion 105 fits into the fastened portion 116. FIGS. 15D and 15E illustrate the state in which the second fastening portion 105 fits into the fastened portion 116. Since the shaft small diameter portion 106 is twisted, the shaft small diameter portion 106 moves to return to the original state, and the first fastening portion 103 and the second fastening portion 105 press the inner wall of the fastened portion 116 at the contact points P₁ and P₂, so that the engaged body 115 does not come off from the holding operation tool 101.

The operator brings the holding operation tool 101 to which the engaged body 115 is attached, to the surgical field, inserts it into the patient's femur, and determines proper alignment by operating the handle 102. When the operator then strikes the top of the handle 102 with a hammer (not shown), the tip projection 107 presses against the bottom surface of the bottom hole 117 so that the engaged body 115 (hip stem) can be press-fitted to the femur. Since the engaged body 115 can be held by the holding operation tool 101 without screwing or the like, the surgery can be swiftly performed. In addition, the risk of screw breakage during hammering is eliminated since no screw coupling is used.

### Eighth Embodiment

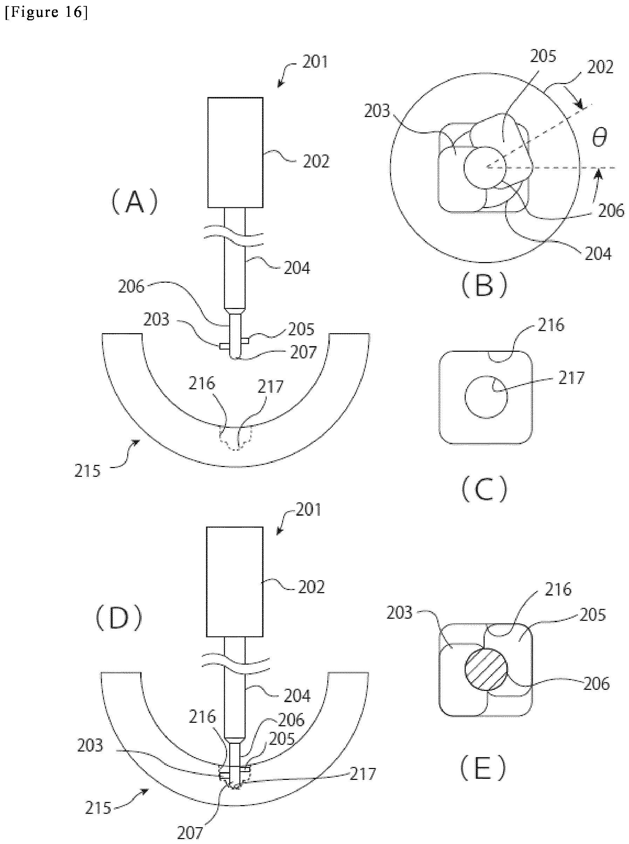

FIG. 16 is a partially schematic cross-sectional view of a holding operation tool for an acetabulum shell in an eighth embodiment of the present invention. As shown in FIG. 16A, a holding operation tool 201 includes a handle 202, a shaft 204, a shaft small diameter portion 206, a first fastening portion 203, a second fastening portion 205, and a tip projection 207. An engaged body 215 is an acetabulum shell. At the bottom of the engaged body 215, a fastened portion 216 and a bottom hole 217 are provided for engaging and driving. As in the example of FIG. 15, the first fastening portion 203 and the second fastening portion 205 are coupled to the shaft small diameter portion 206, that is a shaft connected to the shaft 204.

FIG. 16B is a bottom view of the holding operation tool 201. The first fastening portion 203 and the second fastening portion 205 are shifted by an angle θ from each other, and the second fastening portion 205 cannot fit into the fastened portion 216 shown in FIG. 16C. In the state in which the first fastening portion 203 fits into the fastened portion 216, the handle 202 is twisted to twist the shaft small diameter portion 206 and reduce the angle θ. Then, when the handle 202 is pressed toward the tip side, the second fastening portion 205 fits into the fastened portion 216. FIGS. 16D and 16E illustrate the state in which the second fastening portion 205 fits into the fastened portion 216. Since the shaft small diameter portion 206 is twisted, the shaft small diameter portion 206 moves to return to the original state, and the first fastening portion 203 and the second fastening portion 205 press the inner wall of the fastened portion 216 so that the engaged body 215 does not come off from the holding operation tool 201.

The operator brings the holding operation tool 201 to which the engaged body 215 is attached, to the surgical field, inserts it into a recess of the patent's acetabulum made by reaming with an acetabular reamer, and determines proper alignment by operating the handle 202. When the operator then strikes the top of the handle 202 with a hammer (not shown), the tip projection 207 presses against the bottom surface of the bottom hole 217 so that the engaged body 215 (acetabulum shell) can be press-fitted to the acetabular fossa. Since the engaged body 215 can be held by the holding operation tool 201 without screwing or the like, the surgery can be swiftly performed. In addition, the risk of screw breakage during hammering is eliminated since no screw coupling is used.

### Ninth Embodiment

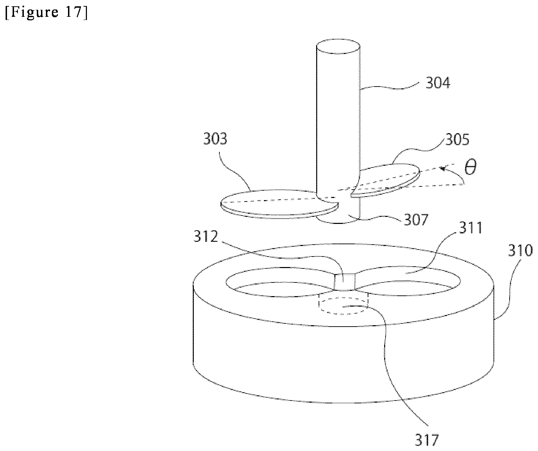

FIG. 17 is a schematic perspective view of a shaft, a fastening portion, and a engaged body in a ninth embodiment of the present invention. A first fastening portion 303 and a second fastening portion 305 are attached to a shaft 304. The first fastening portion 303 is disposed closer to the tip side. The second fastening portion 305 is located above the first fastening portion 303 and is shifted by an angle θ from a position symmetrical to the first fastening portion 303. The shaft 304 has a tip projection 307 at the lower end. A engaged body 310 has a center hole 312 in the upper surface, a recess is formed in a fastened portion 311 shown in the drawing, and a center hole bottom 317 is formed in the center.

In the state in which the first fastening portion 303 fits into the fastened portion 311, the handle is twisted to twist the shaft 304 and reduce the angle θ. Then, when the handle is pressed toward the tip side, the second fastening portion 305 fits into the fastened portion 311. In the present embodiment, although two fastening portions and two corresponding fastened portions are provided, there may be more than two, e.g., six. The number of the first fastening portion and the second fastening portion is not limited to one each. For example, two first fastening portion and one or two second fastening portions may be provided.

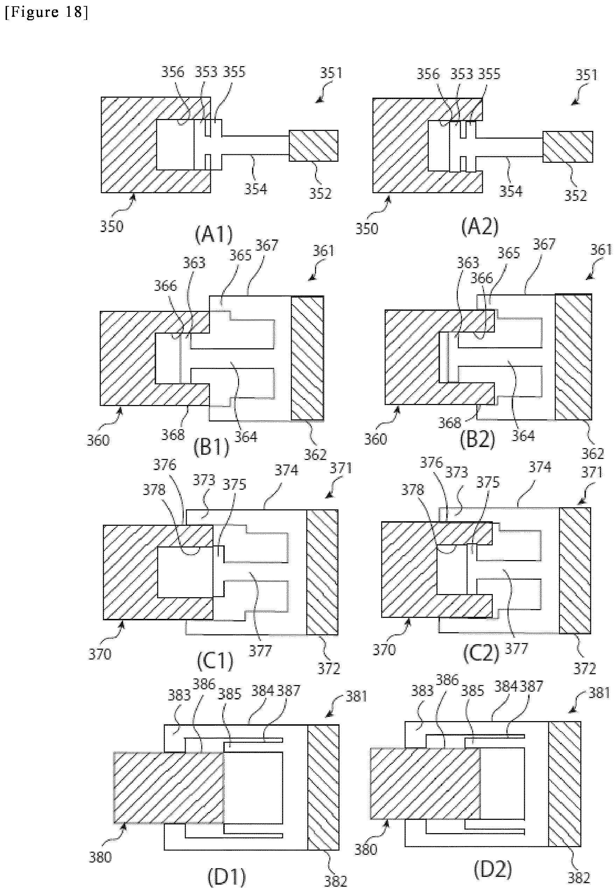

FIG. 18 is a diagram schematically illustrating examples of the relationships between various holding operation tools and various engaged body of the present invention. FIGS. 18A1 and 18A2 illustrate cases where one shaft and one fastened portion are provided, FIGS. 18B1 to 18C2 illustrate cases where two shafts and two fastened portions are provided, and FIGS. 18D1 and 18D2 illustrates cases where two shafts and one fastened portion are provided. These cases are merely examples, and the present invention is not limited to these examples.

Referring to FIG. 18, a holding operation tool 351 includes a handle 352, a shaft 354, a first fastening portion 353, a second fastening portion 355. A engaged body 350 has a fastened portion 356. The first fastening portion 353 fits into the fastened portion 356, but the second fastening portion 355 is shifted in phase and cannot fit into the fastened portion 356 as shown in FIG. 18A1. When the handle 352 is twisted to reduce the phase difference, the second fastening portion 355 fits into the fastened portion 356 so that the holding operation tool 351 can firmly hold the engaged body 350 as shown in FIG. 18A2.

Referring to FIG. 18B1, a holding operation tool 361 includes a handle 362, a first shaft 364, a second shaft 367, a first fastening portion 363, and a second fastening portion 365. A engaged body 360 has a recess 366 and an outer side surface 368. The first fastening portion 363 fits into the recess 366, but the second fastening portion 365 is shifted in phase and cannot engage the outer side surface 368 as shown in FIG. 18B1. When the handle 362 is twisted to reduce the phase difference, the second fastening portion 365 engages the outer side surface 368 so that the holding operation tool 361 can firmly hold the engaged body 360 as shown in FIG. 18B2.

Referring to FIG. 18C1, a holding operation tool 371 includes a handle 372, a first shaft 374, a first fastening portion 373, a second shaft 377, and a second fastening portion 375. An engaged body 370 has a recess 378 and an outer side surface 376. The first fastening portion 373 is engaged with the outer side surface 376, but the second fastening portion 375 is shifted in phase and cannot fit into the recess 378 as shown in FIG. 18C1. When the handle 372 is twisted to reduce the phase difference, the second fastening portion 375 can fit into the recess 378 so that the holding operation tool 371 can firmly hold the engaged body 370 as shown in FIG. 18C2.

Referring to FIG. 18D1, a holding operation tool 381 includes a handle 382, a first shaft 384, a first fastening portion 383, a second shaft 387, and a second fastening portion 385. An engaged body 380 has an outer side surface 386. The first fastening portion 383 is engaged with the outer side surface 386, but the second fastening portion 385 is shifted in phase and cannot be engaged with the outer side surface 386 as shown in FIG. 18D1. When the handle 382 is twisted to reduce the phase difference, the second fastening portion 385 can be engaged with the outer side surface 386 so that the holding operation tool 381 can firmly hold the engaged body 380 as shown in FIG. 18D2.

### Tenth Embodiment

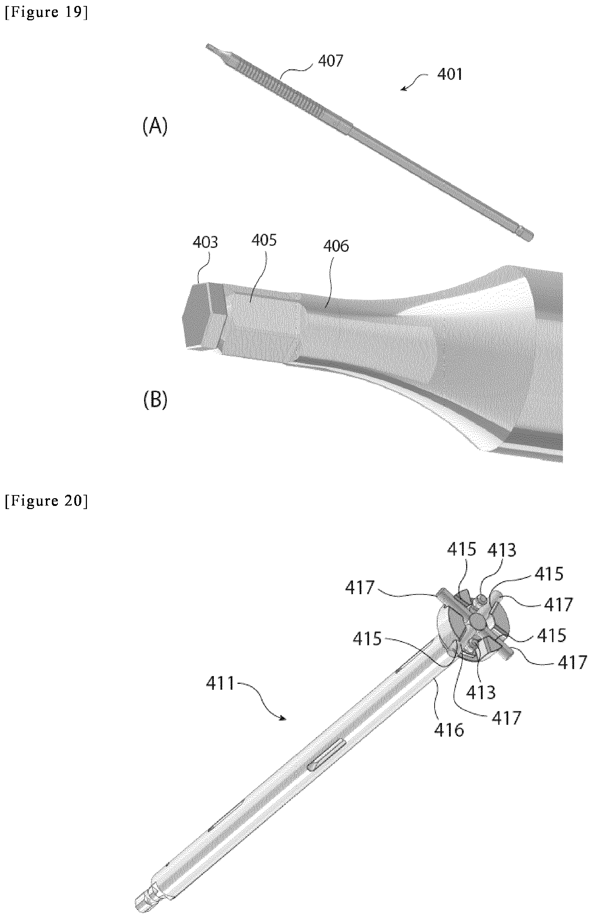

FIG. 19 is a perspective view of a flexible screwdriver of a tenth embodiment. A holding operation tool 401 is a screwdriver for operating a screw and includes a flexible portion 407 on a shank that can be freely changed in direction (see FIG. 19A).

A first fastening portion 403, a second fastening portion 405, and a second shaft 406 are provided at the tip (see FIG. 19B). When the first fastening portion 403 is fitted to the fastened portion of a screw (not shown) and a handle (not shown) is twisted and pressed to fit the second fastening portion 405 to the fastened portion, the screw does not fall from the tip of the flexible screwdriver 401.

### Eleventh Embodiment

FIG. 20 is a perspective view of an acetabular reamer cup holder of an eleventh embodiment. The acetabular reamer cup holder, that is a holding operation tool 411, is a tool for holding an acetabular reamer cup for reaming an acetabulum.

A cross bar 417 is a held portion that is attached to the acetabular reamer cup. The acetabular reamer cup has two holes (not shown) into which two first fastening portions 413 fit. When the first fastening portions 413 are fitted into the two holes and then twisted, the cross bar 417 fits into the recesses in the second fastening portions 415 so that the acetabular reamer cup does not fall out of the acetabular reamer cup holder 411.

The second fastening portions 515 are connected to a second shaft 416, and the first fastening portions 515 are connected to the first fastening portions (not shown) provided in the second shaft 416.

### Twelfth Embodiment

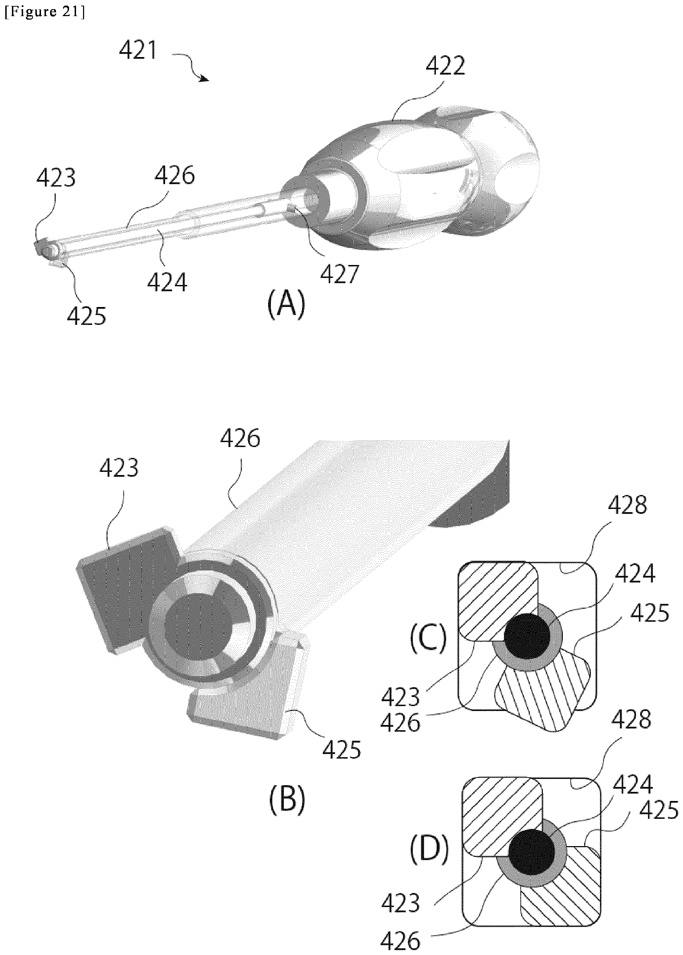

FIG. 21 is a perspective view of a stem holder of a twelfth embodiment. A holding operation tool 421 includes a handle 422, a first shaft 424, and a second shaft 426. The second shaft 426 is coupled to the handle 422, and the first shaft 424 is coupled to the second shaft 426 by a fastening pin 427. The first shaft 424 includes a first fastening portion 423 at the tip, and the second shaft 426 includes a second fastening portion 425 at the tip (see FIG. 21A).

FIG. 21B is an enlarged view of the tip portion.

FIG. 21C illustrates a state in which the first fastening portion 423 fits into a substantially square fastened portion 428 (recess) formed in a hip stem. The second fastening portion 425 is shifted by a predetermined angle as shown in the drawing, and cannot fit into the recess 428.

When the handle 422 is twisted and pressed toward the tip, the second fastening portion 425 fits into the recess 428 (see FIG. 21D) so that the hip stem does not fall off the stem holder 421.

### Thirteenth Embodiment

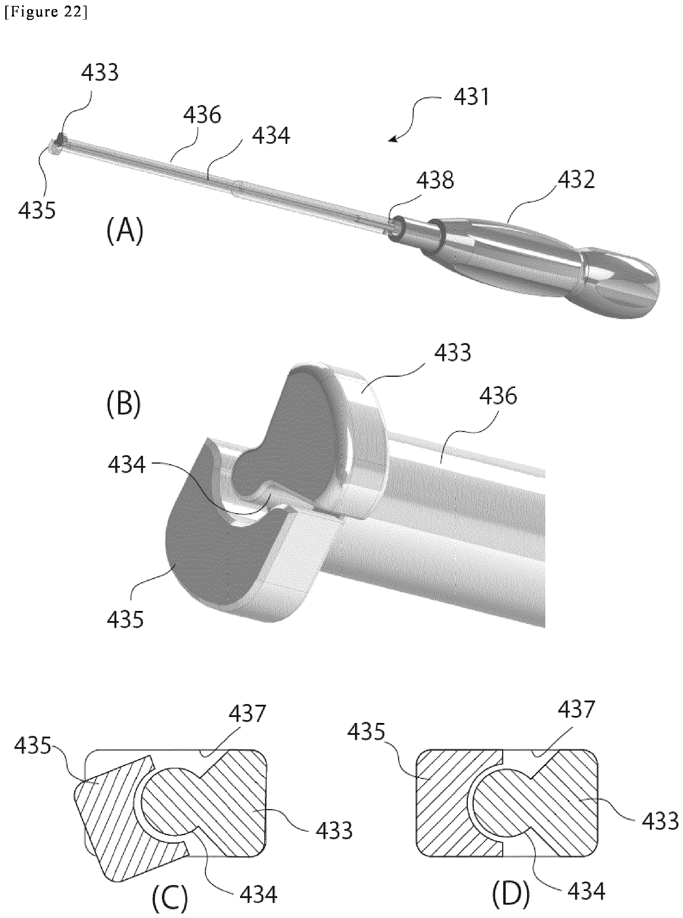

FIG. 22 is a perspective view of a stem holder of a thirteenth embodiment. A holding operation tool 431 includes a handle 432, a first shaft 434, and a second shaft 436. The second shaft 436 is coupled to the handle 432, and the first shaft 434 is coupled to the second shaft 436 by a fastening pin 438. The first shaft 434 includes a first fastening portion 433 at the tip, and the second shaft 436 includes a second fastening portion 435 at the tip (see FIG. 22A).

FIG. 22B is an enlarged view of the tip portion.

FIG. 22C illustrates a state in which the first fastening portion 433 fits into a substantially rectangular fastened portion 437 (recess) formed in a hip stem. The second fastening portion 435 is shifted by a predetermined angle as shown in the drawing, and cannot fit into the recess 437.

When the handle 432 is twisted and pressed toward the tip, the second fastening portion 435 fits into the recess 437 (see FIG. 22D) so that the hip stem does not fall off the stem holder 431.

When the recess 437 is shallow, the arrangement positions of the first fastening portion 433 and the second fastening portion 435 in the axial direction need to be identical. As shown in the drawing, the first fastening portion 433 and the second fastening portion 435 are flush with each other. In this case, the second shaft 436 is slightly inclined to cause the first fastening portion 433 to be slightly caught on the fastened portion 437, so that the first fastening portion 433 and the second fastening portion 435 can fit into the fastened portion 437 at the same time.

### Fourteenth Embodiment

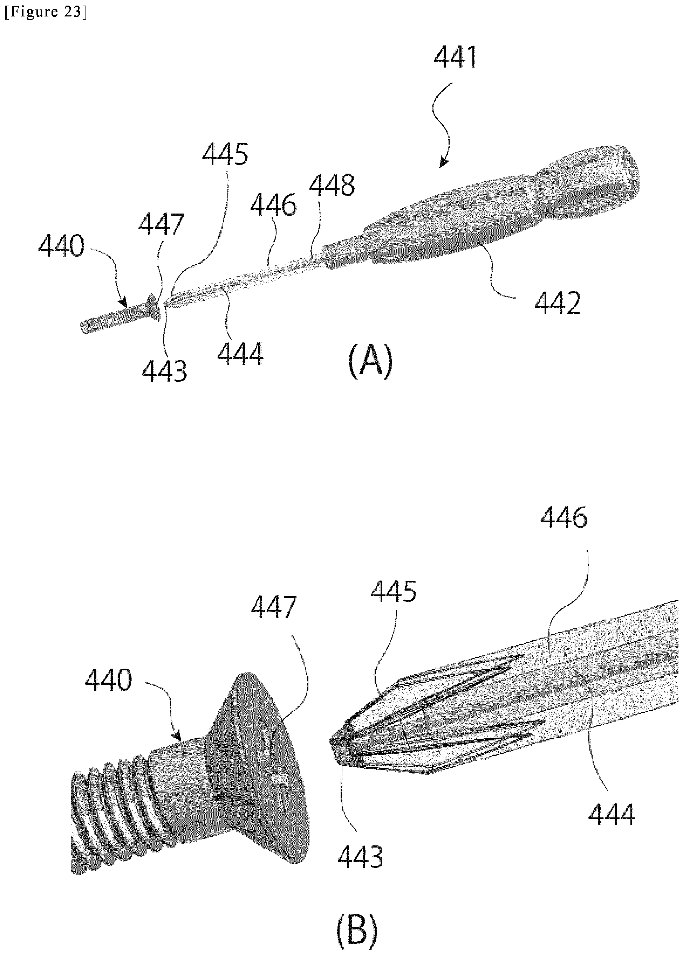

FIG. 23 is a perspective view of a screwdriver of a fourteenth embodiment. A holding operation tool 441 includes a handle 442, a first shaft 444, and a second shaft 446. The second shaft 446 is coupled to the handle 442, and the first shaft 444 is coupled to the second shaft 446 by a fastening pin 448. The first shaft 444 includes a first fastening portion 443 at the tip, and the second shaft 446 includes a second fastening portion 445 at the tip (see FIG. 23A).

FIG. 23B is an enlarged view of a engaged body 440 (screw) and the tip portion of the screwdriver 441. The engaged body 440 has a fastened portion 447 that is a cross groove in the head. The first fastening portion 443 fits into the cross groove 447, but the second fastening portion 445 is shifted in phase by a predetermined angle from the first fastening portion 443 and thus cannot fit into the cross groove 447.

When the handle 442 is twisted and pressed toward the tip, the second fastening portion 445 fits into the cross groove 447 so that the screw 440 does not fall off the screwdriver 441.

### Fifteenth Embodiment

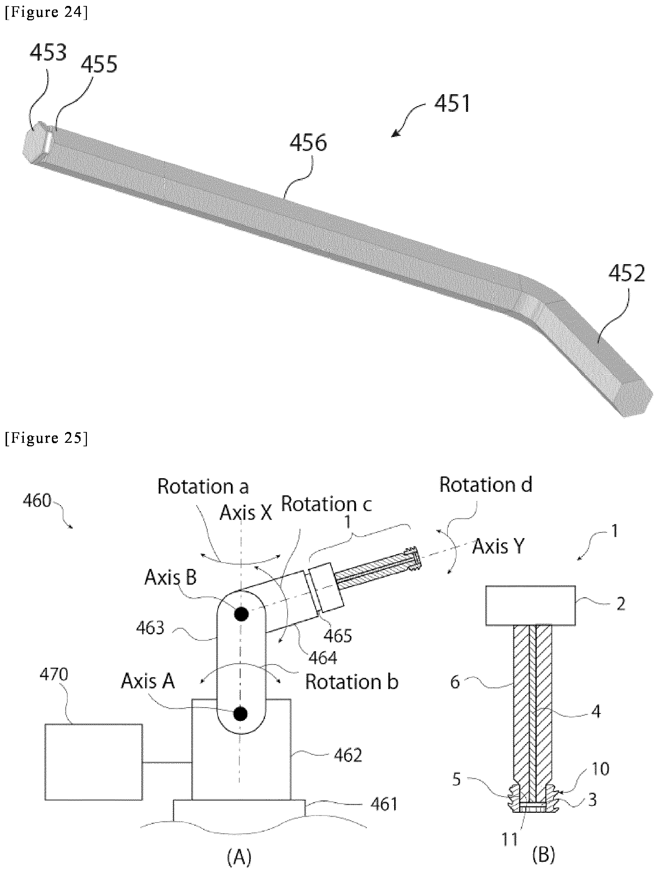

FIG. 24 is a perspective view of a hexagonal wrench of a fifteenth embodiment. A holding operation tool 451 includes a handle 452, a first shaft (not shown), and a second shaft 456. The second shaft 456 is coupled to the handle 452, and the first shaft (not shown) is internally coupled to the second shaft 456. The first shaft includes a first fastening portion 453 at the tip, and the second shaft 456 includes a second fastening portion 455 at the tip.

When operations similar to those described above are performed, the first fastening portion 453 and the second fastening portion 455 are fitted into a hexagonal hole formed in the head of a engaged body (not shown) so that the engaged body (not shown), such as a bolt, does not fall from the hexagonal wrench 451.

### Sixteenth Embodiment

FIG. 25A is a schematic view of a robot of a sixteenth embodiment that includes, at the tip of an arm, the holding operation tool of the first embodiment of the present invention, and FIG. 25B illustrates the holding operation tool 1 attached to a robot 460. The holding operation tool shown in FIG. 25B is the same in structure as the holding operation tool of the first embodiment shown in FIG. 2, and therefore the description thereof is omitted. The robot 460 includes a first arm 462, a second arm 463, a third arm 464, and a rotation shaft 465 connected to a robot main body 461. The first arm 462 through the third arm 464 and the rotation shaft 465 are also collectively referred to as the arm portion.

A controller 470 that controls the arm portion, is connected to the first arm 462 to control the movement of the arm portion. The first arm 462 makes a rotation a about an axis X, the first arm 462 and the second arm 463 are coupled by a support shaft A, and the second arm 463 can make a rotation b about the support shaft A. The second arm 463 and the third arm 464 are coupled by a support shaft B, and the third arm 464 can make a rotation c about the support shaft B. The third arm 464 includes the rotation shaft 465 at the tip, and the rotation shaft 465 can make a rotation d about an axis Y.

The holding operation tool 1 is attached to the tip side surface of the rotation shaft 465, and an engaged body 10 is engaged with the tip of the holding operation tool 1. Even when the arm portion of the robot 460 makes a fast movement, the engaged body 10 does not separate from and fall from the holding operation tool 1, so that it can be used in vehicle assembly lines, surgery-supporting robots, and the like.

### [Industrial Applicability]

As described above, the holding operation tool of the present invention that fastens and operates a engaged body can preferably be used as a holding operation tool that does not pose a risk of the engaged body, such as a screw, falling off.

### [Reference Signs List]

1, 21, 31, 41, 51, 61, 71, 81, 91, 101, 201, 351, 361, 371,381, 401, 411, 421, 431, 441, 451; Holding operation tool
2, 22, 32, 42, 52, 62, 82, 92, 102, 202, 352, 362, 372, 382, 422, 432, 442, 452; Handle
3, 23, 33, 43, 53, 63, 83, 93, 103, 203, 303, 353, 363, 373, 383, 403, 413, 423, 433, 443, 453; First fastening portion
4, 24, 34, 44, 54, 64, 84, 94, 364, 374, 384, 424, 434, 444; First shaft
5, 25, 35, 45, 55, 65, 85, 95, 105, 205, 305, 355, 365, 375, 385, 405, 415, 425, 435, 445, 455; Second fastening portion
6, 26, 36, 46, 56, 66, 86, 96, 367, 377, 387, 406, 416, 426, 436, 446, 456; Second shaft
10, 15, 15A, 115, 215, 310, 350, 360, 370, 380, 440; engaged body

## Claims

1. A holding operation tool that fastens and operates an engaged body including a fastened portion to be fastened, comprising:
a handle that operates the engaged body;
a shaft that protrudes from the handle;
a first fastening portion that is engaged with the fastened portion and is disposed on a tip side of the shaft; and
a second fastening portion that is shaped so as to be incapable of being engaged with the fastened portion when the shaft is merely moved to the tip side after the first fastening portion is engaged with the fastened portion, and so as to be engaged with the fastened portion when the shaft is twisted by a predetermined angle and is moved to the tip side, and that is disposed on the shaft.

2. The holding operation tool according to claim 1, wherein
the shaft has a first shaft that is coupled to the first fastening portion and a second shaft that is coupled to the second fastening portion, and
the first shaft and the second shaft are concentric, one of the first shaft and the second shaft has a cylindrical body through which the other passes, and the both shafts are coupled to the handle.

3. The holding operation tool according to claim 1, wherein
the shaft has a first shaft that is coupled to the first fastening portion and a second shaft that is coupled to the second fastening portion,
the first shaft and the second shaft are concentric, and one of the first shaft and the second shaft has a cylindrical body through which the other passes, and
one shaft of the first shaft and the second shaft is coupled to the handle, and the other shaft is coupled to the one shaft.

4. The holding operation tool according to any one of claims 1 to 3, wherein
the fastened portion is a recess formed in the engaged body, and the first fastening portion and the second fastening portion engage with the recess.

5. The holding operation tool according to any one of claims 1 to 3, wherein
the fastened portion is an outer side surface formed on the engaged body, and the first fastening portion and the second fastening portion are engaged with the outer side surface.

6. The holding operation tool according to any one of claims 1 to 3, wherein
the fastened portion is a recess formed in the engaged body and is an outer side surface formed on the engaged body, one of the first fastening portion and the second fastening portion is engaged with the recess, and the other fastening portion is engaged with the outer side surface.

7. A holding operation tool set comprising:
engaged bodys including a fastened portion to be fastened;
a holding operation tool that fastens and operates the engaged body, the holding operation tool including:
a handle that operates the engaged body;
a shaft that protrudes from the handle;
a first fastening portion that is engaged with the fastened portion and is disposed on a tip side of the shaft; and
a second fastening portion that is shaped so as to be incapable of being engaged with the fastened portion when the shaft is merely biased toward the tip side after the first fastening portion is engaged with the fastened portion, and so as to be engaged with the fastened portion when the handle is turned to twist the shaft by a predetermined angle and bias the shaft toward the tip side, and that is disposed on the shaft; and
a base plate that detachably holds the engaged body.

8. A robot that fastens and operates an engaged body including a fastened portion to be fastened, comprising:
a robot main body; an arm portion that is connected to the robot main body; a controller that controls the arm portion; and a holding operation tool that fastens the engaged body at a tip of the arm portion, wherein
the holding operation tool includes:
a handle that operates the engaged body;
a shaft that protrudes from the handle;
a first fastening portion that is engaged with the fastened portion and is disposed on a tip side of the shaft; and
a second fastening portion that is shaped so as to be incapable of being engaged with the fastened portion when the shaft is merely moved to the tip side after the first fastening portion is engaged with the fastened portion, and so as to be engaged with the fastened portion when the shaft is twisted by a predetermined angle and is moved to the tip side, and that is disposed on the shaft.
